# FASCICULE DE BREVET EUROPEEN

(11) **EP 1 756 677 B1**
(45) Date de publication et mention de la délivrance du brevet: **04.04.2018**
(21) Numéro de dépôt: 05777176.8
(22) Date de dépôt: 10.06.2005
(51) Int. Cl.: G03H 3/00, G01N 29/06, G01N 21/49

(54) **PROCEDE ET INSTALLATION D'IMAGERIE ACOUSTO-OPTIQUE**
VERFAHREN UND EINRICHTUNG FÜR AKUSTOOPTISCHE BILDGEBUNG
METHOD AND INSTALLATION FOR ACOUSTO-OPTIC IMAGING

(30) Priorité: 17.06.2004 FR 0406592
(43) Date de publication de la demande: 28.02.2007
(73) Titulaire: CENTRE NATIONAL DE LA RECHERCHE SCIENTIFIQUE -CNRS-, 75794 Paris Cedex 16 (FR); Sorbonne Université, 75006 Paris (FR)
(72) Inventeur: GROSS, Michel, F-93420 Villepinte (FR); RAMAZ, Francois, Georges, Gérard, F-94270 Le Kremlin-Bicetre (FR); FORGET, Benoît, Claude, F-75005 Paris (FR); ROOSEN, Gérald, F-78720 La Celle Les Bordes (FR); DELAYE, Philippe, F-75010 Paris (FR); BOCCARA, Albert-Claude, F-75004 Paris (FR)
(74) Mandataire: Regimbeau
(86) Numéro de dépôt international: PCT/FR2005/001446
(87) Numéro de publication internationale: WO 2006/005836

(56) Documents cités:
- US-A- 4 284 324
- US-B1- 6 401 540
- SUI L ET AL: "Enhanced detection of acousto-photonic scattering using a photorefractive crystal" PROCEEDINGS OF THE SPIE - THE INTERNATIONAL SOCIETY FOR OPTICAL ENGINEERING SPIE-INT. SOC. OPT. ENG USA - CONFERENCE 25-26 JAN. 2004, vol. 5320, no. 1, 2004, pages 164-171, XP002318021 ISSN: 0277-786X
- GROSS M ET AL: "Shot-noise detection of ultrasound-tagged photons in ultrasound-modulated optical imaging" OPTICS LETTERS OPT. SOC. AMERICA USA, vol. 28, no. 24, 15 décembre 2003 (2003-12-15), pages 2482-2484, XP002318022 ISSN: 0146-9592
- MURRAY T W ET AL: "Adaptive optical array receivers for detection of surface acoustic waves" APPLIED OPTICS OPT. SOC. AMERICA USA, vol. 39, no. 19, 1 juillet 2000 (2000-07-01), pages 3276-3284, XP002318023 ISSN: 0003-6935

## Description

La présente invention est relative aux procédés et installations d'imagerie acousto-optique.

Plus particulièrement, l'invention concerne un procédé d'imagerie acousto-optique.

Dans ce genre de procédé, on illumine un objet à imager avec une source lumineuse de type laser. Par ailleurs, on fait propager des ondes acoustiques dans l'objet avec une source ultra-sonore. On obtient une information pour une zone de l'objet à imager en détectant un signal lié aux propriétés de couplage entre l'onde lumineuse et l'onde ultra-sonore qui fait spécifiquement vibrer la zone en question. En effet, lorsqu'une onde ultra-sonore, de fréquence acoustique fₐ traverse un milieu diffusant (tel que par exemple un tissu biologique, ou autre), elle provoque un ébranlement périodique des diffuseurs et une modulation périodique de l'indice de réfraction du milieu. Si une onde laser, de fréquence incidente f_{I} est diffusée par le milieu, le mouvement des diffuseurs et la modulation de l'indice du milieu génèrent une onde signal comprenant d'une part une composante porteuse (à la fréquence f_{I}) et d'autre part une composante acousto-optique diffusée sur l'une ou l'autre des bandes latérales acoustiques (de fréquence f_{AO} = fₐ ± f_{I}). L'imagerie acousto-optique consiste à déterminer le poids de cette composante à la fréquence f_{AO} en fonction de la position de focalisation de l'onde acoustique dans le milieu diffusant.

Historiquement, la détection a été initialement effectuée en utilisant un détecteur mono-pixel. Néanmoins, cette technique présente une faible sensibilité.

En effet, la détection est effectuée en mesurant les interférences entre deux composantes de l'onde signal : la composante porteuse, à la fréquence f_{I}, et la composante acousto-optique, à la fréquence f_{AO}. Ces deux fréquences étant sensiblement différentes entre elles de la valeur de la fréquence acoustique fₐ de l'onde ultra-sonore, la détection est hétérodyne. Une telle détection n'est efficace que pour une étendue géométrique très réduite, de sorte que la majeure partie du signal est perdue.

D'autre part, du fait de la présence de diffuseurs dans le milieu, les composantes porteuse et acousto-optique de l'onde signal sont deux champs de speckle aléatoires, de sorte que l'information pertinente n'est obtenue qu'en moyennant spatialement et/ou temporellement le signal détecté.

Une amélioration importante a été apportée par l'ESPCI (voir en particulier *"*Ultrasonic tagging of photon paths in scattering media: parallel speckle modulation", Levêque et al., publié dans Optic Letters 24: 181, 1999). Dans ce dispositif, le détecteur mono pixel est remplacé par un détecteur multi pixels tel qu'une caméra CCD. Un problème se pose néanmoins en ce qu'une telle caméra est trop lente pour détecter un signal d'interférence entre les composantes porteuse et acousto-optique de l'onde signal, qui présente une fréquence élevée de l'ordre de celle de l'onde acoustique (quelques MHz, typiquement). Pour détecter un signal, l'ESPCI ne détecte plus les interférences de la composante acousto-optique avec la composante porteuse, mais avec une composante de référence traversant le milieu et obtenue par modulation d'amplitude de l'onde incidente à une fréquence proche de celle de la composante acousto-optique (typiquement à quelques Hz près). On obtient ainsi une interférence entre la composante de référence et la composante acousto-optique qui est suffisamment lente pour être détectée par chaque pixel de la caméra. Pour obtenir l'information relative à la zone vibrante, il faut sommer sur tous les pixels de la caméra le signal détecté.

Cette technique n'est pourtant pas optimale car, d'une part, le signal mesuré comprend une composante importante de bruit dû aux photons simplement diffusés ayant traversé une zone de l'objet à imager ne vibrant pas, et d'autre part, la composante de référence est relativement faible, car elle traverse le milieu diffusant.

En outre, chacun des signaux porte un bruit dit « de décorrélation de speckle ». La lumière, diffusée par le milieu, est émise sous la forme d'une onde de speckle, constituée de grains. D'un grain de speckle à l'autre, l'amplitude et la phase de l'onde signal varient de manière aléatoire. Si, au cours du temps, le milieu diffusant se modifie (c'est en particulier le cas pour les tissus vivants), les diffuseurs changent de position. Cela modifie la position, l'intensité et la phase des grains de speckle (on dit que le speckle se décorrèle).

Le document SUI L ET AL : « Enhanced detection of acoustophotonic scattering using a photorefractive crystal » PROCEEDINGS OF THE SPIE-INT.SOC. OPT.ENG USA - CONFERENCES 25-26 JAN. 2004, vol. 5320, n°1, 2004, pages 164-171 a été publié après la date de priorité de la présente demande, mais correspond aux proceedings d'une conférence qui s'est tenue début 2004 donc avant ladite date de priorité. Ce document décrit un procédé d'imagerie acousto-optique d'un objet comprenant les étapes suivantes :
- génération de la vibration d'une zone de l'objet à l'aide d'une onde acoustique,
- application d'une onde lumineuse sur l'objet et émission d'une onde signal comportant une composante AO décalée en fréquence,
- génération d'une onde pompe,
- enregistrement de la figure d'interférence entre l'onde de référence/pompe et l'onde signal,
- génération et détection de l'onde diffractée par le cristal photorefractif.

En imagerie acousto-optique, on cherche à mesurer l'intensité totale de la composante acousto-optique de l'onde signal. Celle-ci est beaucoup plus faible que celle correspondant à la composante non marquée acoustiquement (la composante porteuse ou la composante de référence, selon la technique utilisée), qui est également vue par le détecteur. Si une partie continue associée à la composante non marquée acoustiquement est facilement éliminable au cours de la détection, les variations d'amplitude et de phase de l'onde signal, qui se traduisent par la décorrélation du speckle, conduisent souvent à un faux signal appelé « bruit de décorrélation de speckle ».

Les tissus biologiques vivants, pour lesquels la technique d'imagerie acousto-optique est amenée à être utilisée, par exemple pour le dépistage du cancer du sein, ou autre, conduisent à un bruit important de décorrélation de speckle. Il est donc préférable de pouvoir effectuer des mesures rapides, ce pour quoi l'utilisation de détecteurs multi-pixels, plutôt lents, n'est pas bien adaptée. Il manque donc un procédé qui puisse garantir une bonne sensibilité de mesure pour les tissus biologiques.

A cet effet, selon l'invention, on prévoit un procédé d'imagerie acousto-optique d'un objet tel que défini en revendication 1 annexée.

Grâce à ces dispositions, on peut s'affranchir du bruit causé par la composante non marquée de l'onde signal, car le réseau d'indice ne se grave que pour un interférogramme de fréquence nulle entre la composante acousto-optique du signal et l'onde pompe. En outre, il est possible d'utiliser un détecteur rapide tel une photodiode lors de la lecture du matériau holographique dynamique.

Dans des modes de réalisation préférés de l'invention, on peut éventuellement avoir recours en outre à l'une et/ou à l'autre des dispositions définies dans les revendications dépendantes.

Selon un autre aspect, l'invention concerne une installation d'imagerie acousto-optique d'un objet tel que defini en revendication 13 annexée.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1 est une vue schématique d'une installation d'imagerie acousto-optique selon l'invention,
- la figure 2 est un diagramme temporel représentant une modulation de phase de l'onde acoustique,
- les figures 3a et 3b sont un diagramme de phase représentant les amplitudes des différentes composantes dans un cristal photo réfractif respectivement lors de l'écriture et de la lecture du cristal,
- la figure 4 représente un signal obtenu sur un écran d'oscilloscope pour deux objets à imager, et
- les figures 5a et 5b sont des vues schématiques de deux variantes de réalisation d'un dispositif de génération d'ondes lumineuses.

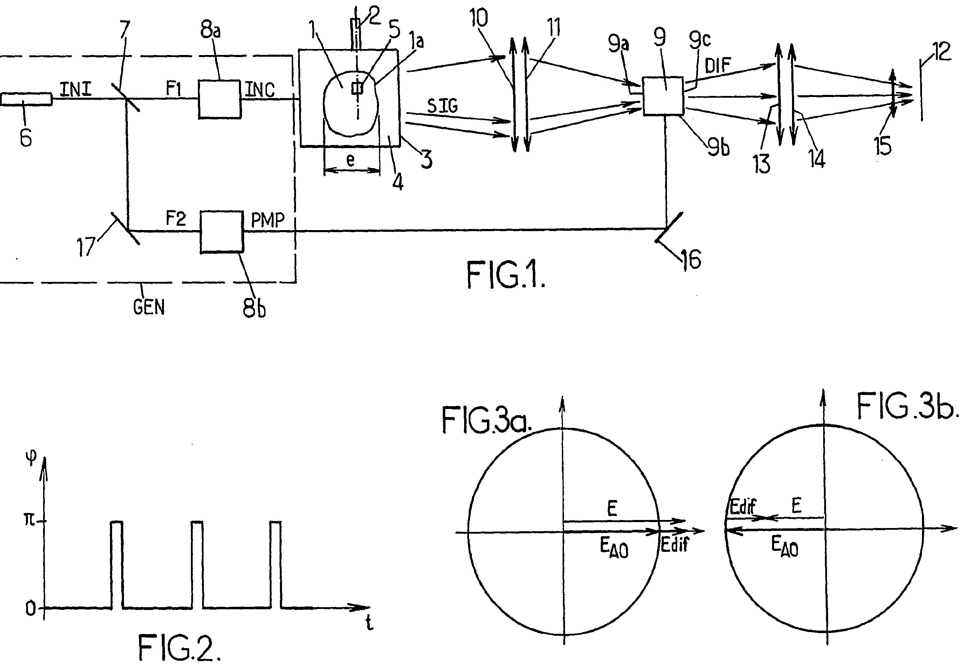

La figure 1 représente schématiquement une installation d'imagerie acousto-optique susceptible d'être utilisée dans le cadre de l'invention.

On dispose d'un objet 1 à imager à l'aide de ladite installation. Cet objet est un milieu diffusant, typiquement de quelques centimètres d'épaisseur e et peut être par exemple un tissu biologique, tel qu'une partie de corps humain ou animal, ou autre.

Un transducteur piézo-électrique 2 est en contact acoustique avec l'objet 1, soit directement en contact, soit par exemple couplé acoustiquement à l'objet 1 par l'immersion de celui-ci dans une cuve 3 remplie d'eau 4. On utilise par exemple un transducteur piézo-électrique Panamétrics de diamètre 37 mm présentant une face de sortie sphérique de rayon 75 mm. Le transducteur fait vibrer la zone de l'objet en regard de laquelle il est, et en particulier la zone 5 à la fréquence acoustique fₐ ultra-sonore, telle que par exemple 2 MHz. Le transducteur piézo-électrique 2 est placé en regard d'une position donnée de la surface de l'objet. Le transducteur piézo-électrique 2 est en outre adapté pour focaliser sur une zone donnée 5 de l'objet à imager dont on veut obtenir une information optique.

On dispose en outre de moyens de commande (non représentés), de type micro-ordinateur, ou autre, adaptés pour changer la distance focale du transducteur piézoélectrique 2 et pour commander le déplacement de celui-ci en regard de la surface de l'objet à imager 1, de manière à scanner dans une, deux ou trois dimensions l'objet à imager 1.

On dispose en outre d'un dispositif de génération d'onde lumineuses GEN adapté pour générer deux ondes lumineuses cohérentes décalées en fréquence environ de la valeur de la fréquence acoustique fₐ imprimée par le transducteur piézo-électrique 2 à la zone 5 de l'objet à imager 1. D'autres types de dispositifs que celui décrit par la suite peuvent également être utilisés à cette fin.

On dispose d'un laser 6, qui est par exemple un laser YAG monofréquence de longueur d'onde 1,06 µm et de puissance 100 mW. Le laser 6 émet une onde optique initiale INI présentant dans l'exemple considéré, une polarisation verticale. L'onde initiale INI est séparée en un premier faisceau F1 et un deuxième faisceau F2 par un prisme séparateur 7, par exemple un prisme séparateur 50 % par lequel la puissance disponible sur chaque faisceau F1, F2 est d'environ 50 mW.

On utilise en outre un premier modulateur acousto-optique 8a et un deuxième modulateur acousto-optique 8b recevant respectivement en entrée les faisceaux F1 et F2. Ces modulateurs acousto-optique 8a et 8b sont par exemple des modulateurs de la Société Cristal Technology constitués d'une cellule acousto-optique de Dioxyde de Tellure (TeO₂) animée par une horloge radio fréquence sinusoïdale de fréquence df, transmettant, à partir de l'onde qui lui est appliquée, à la fois un faisceau non diffracté et un faisceau diffracté décalé en fréquence de la valeur df par rapport à l'onde qui lui est appliquée. A titre d'exemple, le premier modulateur acousto-optique 8a est animé par une horloge de fréquence df = 73 MHz et délivre en sortie une onde que nous appellerons par la suite l'onde incidente INC décalée en fréquence de 73 MHz par rapport à l'onde initiale INI. Le deuxième modulateur acousto-optique 8b est de conception similaire et est animé à une fréquence de 75 MHz et délivre en sortie d'onde que nous appellerons par la suite onde pompe PMP décalée en fréquence de 75 MHz par rapport à l'onde initiale INI. En utilisant des modulateurs acousto-optique 8a, 8b d'efficacité environ 50 %, on dispose ainsi d'une puissance environ 25 mW pour chacune des ondes incidentes INC et pompe PMP dans l'exemple considéré.

On génère ainsi deux ondes lumineuses cohérentes entre elles décalées en fréquence d'une valeur environ égale à la fréquence acoustique appliquée par le transducteur piézo-électrique 2 à l'objet à imager 1.

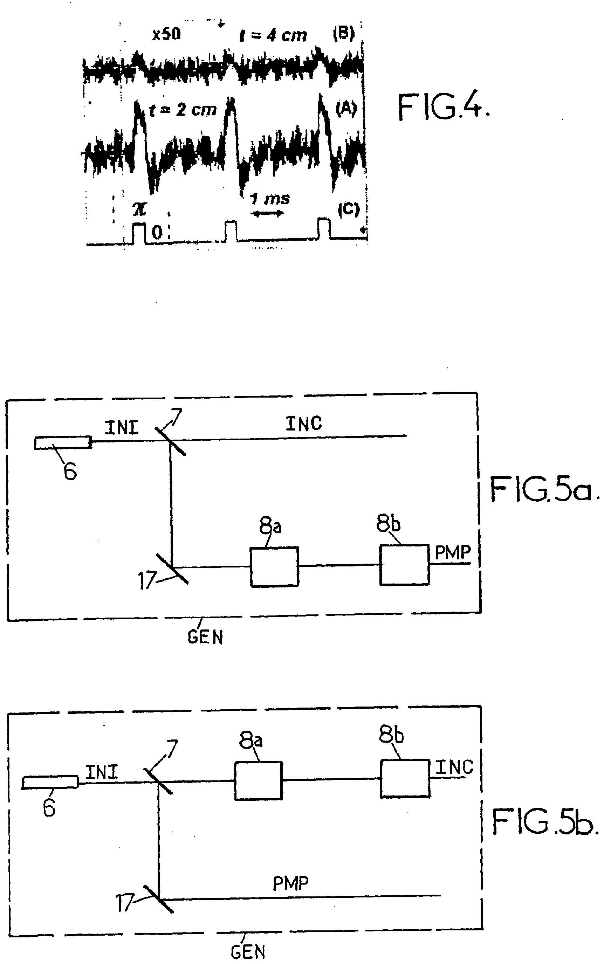

Selon une première variante du dispositif de génération d'ondes lumineuses GEN, représentée sur la figure 5a, l'onde incidente INC ne passe par aucun modulateur acousto-optique, tandis que l'onde pompe est décalée en fréquence, par rapport à l'onde incidente, d'une première fréquence df₁ = - 73 Mhz par le premier modulateur acousto-optique 8a, puis décalée d'une deuxième fréquence df₂ = + 75 Mhz par le deuxième acousto-optique 8b, de sorte que df₁ + df₂ ≅ fₐ.

Selon une deuxième variante du dispositif de génération d'ondes lumineuses GEN, représentée sur la figure 5b, l'onde incidente INC est décalée en fréquence, par rapport à l'onde initiale, d'une première fréquence df₁ = + 73 Mhz par le premier modulateur acousto-optique 8a, puis d'une deuxième fréquence df₂ = - 75 Mhz par le deuxième modulateur acoustique 8b, et l'onde pompe PMP n'est pas décalée en fréquence par rapport à l'onde initiale INI, de sorte que f_{INI} + df₁ + df₂ + fₐ ≅ f_{PMP}, où f_{INI} est la fréquence de l'onde initiale et f_{PMP} la fréquence de l'onde pompe.

Ainsi, selon les variantes des figures 1, 5a et 5b de réalisation d'un dispositif de génération GEN, un premier dispositif de décalage, interposé entre le laser 6 et l'objet à imager 1, comprend un, zéro ou deux modulateurs acousto-optiques, et un deuxième dispositif de décalage, interposé entre le laser 6 et le matériau holographique 9, comprend, respectivement, un, deux, ou zéro modulateurs acousto-optiques. Ainsi, un « dispositif de décalage », au sens de la présente description peut, le cas échéant et dans le cas précité, ne comporter aucun élément.

La suite de la description est donnée pour l'exemple de réalisation du dispositif de génération de la figure 1.

L'onde incidente INC issue du premier modulateur acousto-optique 8a est appliquée sur l'objet à imager 1 en un point quelconque, pas nécessairement lié à la position dans l'objet à imager 1 de la zone 5. La lumière est diffusée par l'objet 1 et génère une onde signal SIG s'étendant dans un large angle solide à l'arrière de l'objet imagé 1 par rapport à la direction d'application de l'onde incidente INC. Certains photons de l'onde incidente sont diffusés par l'objet à imager 1 sans traverser la zone vibrante alors que d'autres photons traversant la zone vibrante sont soumis à l'effet acousto-optique. Par conséquent, l'onde signal SIG comporte principalement deux composantes : d'une part, la composante « porteuse », à la fréquence f_{I} de l'onde incidente INC, qui correspond aux photons ne traversant pas la zone vibrante, et d'autre part, une composante acousto-optique de fréquence f_{AO} = f_{I} ± fₐ correspondant aux photons traversant la zone 5 de l'objet à imager 1. C'est cette composante acousto-optique qui porte l'information relative à la zone 5 de l'objet à imager 1 que l'on cherche à mesurer.

A cet effet, on dispose, selon l'invention, d'un matériau holographique dynamique 9 tel qu'un cristal photo réfractif par exemple en arsenure de gallium (AsGa) de dimensions 1 cm x 1 cm x 1 cm. De tels cristaux photo réfractifs, par exemple décrits dans le brevet US 5,131,748, sont des matériaux holographiques dans lesquels l'interférence d'une onde signal et d'une onde pompe forme un réseau d'indice de réfraction. Ce réseau d'indice de réfraction peut présenter une composante statique en fonction des caractéristiques des ondes.pompe et signal, et un tel réseau statique d'indice de réfraction diffracte l'onde pompe appliquée au cristal photo réfractif pour former une onde diffractée DIF. En tant que cristal photo réfractif 9, on pourrait également utiliser un matériau du type sillénite tel que du BSO, BGO ou BTO, un phosphure d'indium (InP) dopé au fer, un tellurure de cadmium (CdTe) dopé au vanadium, un cristal d'hypothio diphosphate d'étain (SPS), ou tout autre matériau adapté.

A la place du cristal photo réfractif, on pourrait également utiliser à titre de matériau holographique dynamique, un milieu laser inversé tel que YAG ou titane-saphir. Dans un tel matériau, soumis au pompage par un laser adapté, se produit une inversion de population permettant d'y graver un réseau d'indice complexe sous forme d'un réseau de gain. Ces matériaux sont en particulier intéressants car sensibles à des longueurs d'onde comprises entre 770 nm et 1 µm, qui sont des longueurs d'onde utiles en biologie.

Dans l'exemple présenté, l'onde signal SIG est appliquée à une première face 9a du cristal photo réfractif 9. Par exemple, à l'aide d'un premier dispositif optique formé de deux lentilles 10, 11, de grande ouverture, on forme sur la face 9a du cristal photo réfractif 9 une image de la face « arrière » 1a de l'objet à imager 1. On place les lentilles 10 et 11 de manière à récupérer la plus grande partie possible de l'onde SIG, diffusée dans un large angle solide, sur la face 9a du cristal photo réfractif.

L'onde pompe PMP générée par le dispositif générateur GEN (par exemple, l'onde issue du deuxième modulateur acousto-optique 8b) est appliquée au cristal photo réfractif 9, par exemple, sur une face 9b du cristal orthogonale à la face 9a à l'aide d'un deuxième dispositif optique tel qu'une lame réfléchissante 16.

L'interférence entre l'onde signal SIG et l'onde pompe PMP produit un interférogramme en volume à l'intérieur du cristal photo réfractif 9. Cet interférogramme comporte, d'une part, une composante liée à la composante porteuse de l'onde signal et, d'autre part, une composante liée à la composante acousto-optique de l'onde signal. Pour un décalage en fréquence entre l'onde incidente INC et l'onde pompe PMP égal à la fréquence fₐ de l'onde acoustique, la composante de l'interférogramme associée à la composante acousto-optique de l'onde signal SIG (de fréquence f_{I} ± fₐ) est statique puisque la fréquence de l'onde pompe PMP est égale à la fréquence de cette composante acousto-optique de l'onde signal. Par contre, la composante de l'interférogramme formé entre l'onde incidente et l'onde pompe donne une modulation temporelle à la fréquence acoustique. Cette variation est trop rapide pour que soit inscrit un réseau d'indice dans le milieu holographique dynamique. Ainsi, par effet photo réfractif, l'interférogramme statique associé à la composante acousto-optique crée, dans le cristal, un réseau d'indice qui reproduit la figure de speckle associé à ladite composante acousto-optique qui est générée par la vibration de l'échantillon à la fréquence acoustique fₐ, principalement dans la zone 5, et qui caractérise donc les propriétés optiques de l'échantillon dans cette zone.

Simultanément ou ultérieurement, l'onde pompe appliquée au cristal photo réfractif 9 est diffractée par le réseau d'indice formé dans le cristal, dans la même direction que l'onde signal SIG. Comme le réseau d'indice enregistre la structure spatiale de la composante acousto-optique de l'onde signal SIG, l'onde diffractée DIF par le cristal a la même structure spatiale que celle-ci. L'interférence entre l'onde signal SIG et l'onde diffractée DIF reste donc cohérente spatialement avec elle-même sur toute la surface du cristal 9. Il est ainsi possible d'observer cette interférence avec un détecteur mono pixel 12, telle qu'une photo diode en silicium. On peut utiliser un troisième dispositif optique formé des lentilles 13, 14 de grande ouverture pour former une image de la face 9c sur une photo diode 12 distante. Si la photo diode est beaucoup plus petite que la superficie de la face 9c du cristal 9, on peut utiliser une lentille 15 concentrant la lumière sur la photo diode 12.

En appliquant l'onde signal et l'onde pompe sur deux faces orthogonales du matériau holographique dynamique, on limite l'influence, au niveau du signal détecté, des imperfections de montage de l'installation et du cristal, qui ne sont pas diffusées vers le détecteur, mais dans la direction de l'onde pompe.

On peut également choisir d'augmenter la vitesse d'établissement de l'effet photo réfractif dans le cristal en diminuant l'angle entre l'onde signal et l'onde pompe, tout en gardant un angle non nul. On peut ainsi appliquer les deux ondes sur la même face de cristal. Néanmoins, l'influence des imperfections du montage et du cristal sera plus sensible au niveau du détecteur. On pourra également choisir un angle intermédiaire permettant un bon compromis entre ces deux options.

L'intensité lumineuse ainsi détectée au niveau de la photo diode 12 permet d'obtenir une information concernant la zone 5 de l'objet à imager 1, et en déplaçant la focale du transducteur piézo-électrique 2, ou le transducteur lui-même à la surface de l'objet 1, on peut former une image mono, bi, ou tridimensionnelle de l'objet. Cette image peut en outre être couplée à une image par ultrasons (échographie) formée simultanément de l'objet 1, car le transducteur piézo-électrique 2 utilisé peut tout à fait être du type de ceux utilisés pour générer une telle image ultrasonore.

Dans le cadre de l'invention, il est encore possible d'améliorer la qualité de détection au niveau du détecteur 12, comme décrit par la suite.

L'utilisation d'un cristal photo réfractif 9 permet d'éliminer le bruit dû à la composante non marquée de l'onde signal parce que l'effet photo réfractif qui a lieu dans le cristal est sélectif, et ne se produit que pour la composante acousto-optique de l'onde signal SIG. En effet, l'onde pompe PMP est à la même fréquence que la composante acousto-optique. Dans ces conditions, seule l'interférence entre l'onde pompe PMP et la composante acousto-optique de l'onde signal SIG reste stable au cours du temps, et se trouve donc susceptible de générer un réseau d'indice dans le cristal 9. Ce signal d'interférence statique est difficile à distinguer du fond continu parasite dû par exemple au courant d'obscurité de la photo diode, aux impuretés du cristal photo réfractif, ou autre. Pour rendre l'interférence visible par rapport à ce fond continu parasite, il est préférable d'avoir un interférogramme variant dans le temps qui conduise à une modulation temporelle du signal obtenu sur la photo diode 12. A cet effet, on peut moduler en amplitude ou en phase l'une des ondes qui sont impliquées dans la génération de l'interférogramme.

Par exemple, on peut moduler en phase l'onde incidente INC sur l'objet à imager 1, l'onde pompe PMP générée sur la face 9b du cristal photo réfractif 9, ou l'onde acoustique générée par le transducteur piézo-électrique 2. Ces trois options sont possibles, et on décrit par la suite, à titre d'exemple, la modulation en phase de l'onde acoustique.

Pour obtenir une modulation temporelle du signal sur la photo diode, la modulation de l'onde acoustique doit obéir aux trois conditions suivantes :
- la modulation doit être plus rapide que le temps nécessaire à graver le réseau d'indice dans le cristal photo réfractif 9 (afin que le réseau d'indices, qui effectue une moyenne, ne soit pas affecté par la modulation),
- la modulation doit être de bonne amplitude (ce qui permet une bonne sensibilité de détection), et
- l'onde modulée ne doit pas présenter, pendant le temps nécessaire à graver le réseau d'indice, une valeur moyenne nulle (afin que le réseau d'indices, qui dépend de la valeur moyenne de l'onde signal SIG, puisse exister).

De nombreuses modulations sont susceptibles de remplir ces conditions. A titre d'exemple, on représente sur la figure 2 une modulation remplissant ces critères. La phase ϕ acoustique est modulée en créneaux rectangulaires de ϕ = 0 à ϕ = π. Le rapport cyclique des créneaux rectangulaires est pris différent de 1/2, et est par exemple pris égal à un 1/8 pour une fréquence de créneaux de 302 Hz.

La composante acousto-optique de l'onde signal SIG est liée à l'onde acoustique appliquée par le transducteur 2. Par conséquent, la phase de la composante acousto-optique de l'onde signal présente la même modulation de phase que l'onde acoustique. La composante acousto-optique de l'onde signal présente alors une valeur moyenne non nulle égale aux 6/8 de la valeur de la composante acousto-optique de l'onde signal lors d'un déphasage ϕ = 0. Le réseau d'indices gravé dans le cristal photo réfractif 9 est non nul, et correspond à la valeur moyenne <E_{AO}> de la composante acousto-optique de l'onde signal SIG.

Dans l'exemple considéré, le cristal photo réfractif 9 présente des faces 9a, 9b, 9c orientées de manière à ce que le cristal 9 présente une configuration dite de « transfert d'énergie ». Dans cette configuration, l'onde diffractée DIF a la même polarisation que la composante acousto-optique de l'onde signal. De plus, l'onde diffractée DIF est en phase avec <E_{AO}>, c'est-à-dire en phase avec E_{AO} (ϕ = 0).

La modulation de phase ϕ de l'onde acoustique se traduit par une modulation du signal |E|² vu par la photo diode 12, qui est illustrée sur les figures 3a et 3b.

Sur la figure 3a, lorsque la modulation de phase ϕ est égale à 0 (étape d'« écriture »), l'onde diffractée DIF est en phase avec la composante acousto-optique de l'onde signal. L'amplitude E dans le cristal 9 est maximale et correspond à la somme des amplitudes E_{dif} et E_{AO} respectivement de l'onde diffractée et de la composante acousto-optique de l'onde signal. Le signal vu par la photo diode, qui est proportionnel à l'intensité 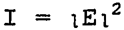 est également maximal.

Sur la figure 3b, lorsque la modulation de phase ϕ est égale à n (étape de « relecture »), l'onde diffractée DIF est en opposition de phase avec la composante acousto-optique de l'onde signal. L'amplitude totale E, correspondant maintenant à la soustraction de l'amplitude de la composante acousto-optique de l'onde signal E_{AO} et de l'amplitude de l'onde diffractée E_{dif} est minimale, ainsi que le signal vu par la photo diode.

La figure 4 présente un écran d'oscilloscope sur lequel le signal (C) représente la modulation de phase de l'onde acoustique appliquée à l'objet à imager, présentant un rapport cyclique de 1/8 et une fréquence de 305 Hz, le signal (A) représente le signal détecté pour un objet à imager qui est un morceau de blanc de poulet de 2 cm d'épaisseur, et le signal (B) représente le signal détecté moyenné, grossi 50 fois, pour un morceau de blanc de poulet de 4 cm d'épaisseur.

Pour d'autres configurations, comme par exemple dans le cas d'un milieu laser pompé, les rôles peuvent être inversés. On a alors opposition de phase pour ϕ=0 et signaux en phase pour ϕ=π.

L'information relative à la zone 5 de l'objet à imager 1 peut être extraite par détection synchrone du signal détecté par la photo diode 12.

Le cristal photo réfractif 9 n'est pas nécessairement utilisé en configuration de « transfert d'énergie ». Il pourrait également être utilisé en configuration de « configuration anisotrope », en utilisant un cristal présentant des orientations différentes des faces 9a, 9b. La composante acousto-optique de l'onde signal et l'onde diffractée par le réseau d'indices sont alors de polarisations orthogonales. Pour faire interférer des deux ondes, on peut par exemple utiliser une lame quart d'onde et un cube polariseur, de manière connue. Un exemple de modulation de phase utilisable dans cette configuration est d'utiliser une modulation de phase en créneaux de 0 à n/2 avec un rapport cyclique de 50 %. On peut ainsi obtenir une détection linéaire.

L'installation ici décrite permet d'obtenir des images de bonne qualité, en particulier parce qu'elle permet de réaliser la détection sur une grande étendue optique avec une photo diode, ce qui n'était pas réalisé par les dispositifs de l'art antérieur.

La sélectivité en fréquence de l'effet photo réfractif est caractérisée par le temps Tp nécessaire pour graver le réseau d'indice, et par l'écart de fréquence fₐ = 2 MHz entre la composante porteuse de l'onde signal et l'onde pompe PMP. Les photons associés à la composante de l'onde signal ne créent pas de réseau d'indice lorsque Tp x fₐ >> 1.

Cette condition de sélectivité est très largement réalisée. Dans l'exemple ci décrit, on a par exemple Tₚ = 1 à 10 ms, ce qui correspond à T_{P} x fₐ = 10³ à 10⁴.

Une telle installation permet d'augmenter la vitesse de mesure par rapport aux installations connues de l'art antérieur. Pour augmenter la vitesse de mesure, on est amené à diminuer le temps de mesure, et donc le temps de gravage Tp. Ce temps de gravage peut être facilement réduit en augmentant la puissance de l'onde pompe PMP par rapport à l'exemple proposé. Par exemple, en augmentant la puissance de l'onde initiale INI, on augmente à la fois la puissance de l'onde incidente INC et la puissance de l'onde pompe PMP. D'une part, une augmentation de la puissance de l'onde pompe PMP réduit le temps de gravage. D'autre part, une augmentation de la puissance de l'onde incidente INC augmente le signal. Par rapport à l'exemple proposé, on peut notablement augmenter la puissance de l'onde initiale, et donc la puissance de l'onde incidente, tout en restant dans les limites de sécurité imposées pour le traitement de tissus humains, la puissance de l'onde pompe ne pose pas de problèmes de sécurité, puisque l'onde pompe ne passe pas par l'échantillon.

Par rapport à l'exemple présenté, on pourra, lors de variantes de réalisation, mettre en oeuvre l'une et/ou l'autre des dispositions suivantes :
- utiliser un laser plus puissant,
- utiliser des lentilles 10, 11, 13, 14 de plus grande ouverture permettant de conserver la même ouverture angulaire avec une surface de diode plus grande,
- utiliser une photo diode 12 de bruit inférieur, telle qu'une photo diode refroidie par effet Peltier,
- utiliser une photo diode de plus grande surface.

Toutes ces dispositions permettraient d'augmenter le rapport signal/bruit de la détection.

## Revendications

1. Procédé d'imagerie acousto-optique d'un objet comprenant les étapes au cours desquelles :
(a) on génère la vibration d'une zone (5) de l'objet (1) en appliquant sur cette zone de l'objet une onde acoustique présentant une certaine fréquence acoustique,
(b) on appliquée audit objet une onde lumineuse incidente (INC) générant ainsi une onde lumineuse signal (SIG) comportant au moins une composante acousto-optique décalée en fréquence par ladite onde acoustique,
(c) on génère une onde pompe (PMP) lumineuse, cohérente avec ladite onde incidente (INC) à une fréquence similaire à la fréquence de ladite composante acousto-optique,
(d) on grave un réseau d'indice complexe dans un matériau holographique dynamique (9) par l'interférence entre l'onde signal et l'onde pompe en appliquant audit matériau ladite onde signal et ladite onde pompe, et
(e) on obtient un paramètre numérique relatif à l'intensité lumineuse en cette zone à partir dudit réseau d'indice complexe
dans lequel, au cours de l'étape (e),
(e1) on génère une onde diffractée par application de ladite onde pompe (PMP) sur ledit réseau d'indice complexe, et
(e2) on détecte l'interférence entre ladite onde diffractée et ladite onde signal à l'aide d'un photo détecteur (12).

2. Procédé selon la revendication précédente, dans lequel on applique une modulation de phase, à une onde choisie parmi l'onde acoustique, l'onde pompe (PMP), et l'onde incidente (INC).

3. Procédé selon la revendication 2, dans lequel ladite modulation de phase est appliquée à l'onde acoustique.

4. Procédé selon la revendication 1, dans lequel on applique une modulation d'amplitude à une onde choisie parmi l'onde acoustique, l'onde pompe (PMP) et l'onde incidente (INC).

5. Procédé selon la revendication 4, dans lequel ladite modulation d'amplitude est appliquée à l'onde acoustique.

6. Procédé selon l'une quelconque des revendications précédentes, dans lequel, on génère l'onde incidente et l'onde pompe en
- générant une onde lumineuse initiale (INI) à l'aide d'un laser,
- séparant ladite onde initiale en un premier faisceau (F1) et un deuxième faisceau (F2),
- décalant la fréquence dudit premier faisceau d'un premier décalage en fréquence pour générer l'onde incidente (INC),
- décalant la fréquence dudit deuxième faisceau d'un deuxième décalage en fréquence pour générer l'onde pompe (PMP),
ledit deuxième décalage en fréquence étant sensiblement égal à la somme du premier décalage en fréquence et de la fréquence acoustique.

7. Procédé selon la revendication 6, dans lequel l'un desdits premier et deuxième décalages en fréquence est nul.

8. Procédé selon l'une quelconque des revendications précédentes dans lequel, au cours de l'étape (d), on applique ladite onde signal sur une première face (9a) dudit matériau holographique dynamique et ladite onde pompe sur une deuxième face (9b) dudit matériau, distincte de la première face.

9. Procédé selon l'une quelconque des revendications 1 à 7, dans lequel, au cours de l'étape (d), on applique ladite onde signal et ladite onde pompe sur une même face du matériau holographique dynamique.

10. Procédé selon l'une des revendications précédentes, dans lequel ledit matériau holographique dynamique est un cristal photo réfractif adapté pour fonctionner en mode « transfert d'énergie ».

11. Procédé selon l'une des revendications 1 à 9, dans lequel ledit matériau holographique dynamique est un cristal photo réfractif adapté pour fonctionner en mode « diffraction anisotrope ».

12. Procédé selon l'une quelconque des revendications précédentes, au cours duquel on obtient en outre une information numérique relative à une deuxième zone de l'objet en appliquant les étapes suivantes :
(a') on génère la vibration de ladite deuxième zone en appliquant à l'objet au niveau de ladite deuxième zone une onde acoustique à une fréquence acoustique, et
on répète les étapes (b) à (e) pour ladite deuxième zone.

13. Installation d'imagerie acousto-optique d'un objet comprenant
(A) un transducteur (2) adapté pour générer la vibration d'une zone (5) de l'objet (1) en appliquant à cette zone de l'objet une onde acoustique présentant une fréquence acoustique,
(B) un dispositif de génération d'ondes lumineuses adapté pour appliquer audit objet une onde lumineuse incidente (INC) et, générant ainsi une onde lumineuse signal (SIG) comportant au moins une composante acousto-optique décalée en fréquence par ladite onde acoustique, et adapté en outre pour générer une onde pompe (PMP) lumineuse, cohérente avec ladite onde incidente à une fréquence similaire à la fréquence de ladite composante acousto-optique,
(C) un matériau holographique dynamique (9) adapté pour qu'y soit gravé un réseau d'indice complexe par l'interférence entre l'onde signal et l'onde pompe, grâce à l' application audit matériau de ladite onde signal et de ladite onde pompe, et
(D) un dispositif de détection adapté pour obtenir un paramètre numérique relatif à l'intensité lumineuse en cette zone à partir dudit réseau d'indice complexe,
dans laquelle ledit dispositif de détection (E) comprend un détecteur (12) adapté pour détecter l'interférence entre une onde diffractée générée par ladite onde pompe traversant ledit réseau d'indice complexe, et ladite onde signal.

14. Installation selon la revendication 13, dans laquelle ledit détecteur est une photo diode monopixel.

15. Installation selon la revendication 13 ou la revendication 14 comprenant
un premier dispositif optique (10, 11) adapté pour appliquer l'onde signal sur une première face du matériau holographique dynamique,
un deuxième dispositif optique (16) adapté pour appliquer l'onde pompe sur une deuxième face du matériau photo réfractif, et
un troisième dispositif optique (13, 14, 15) adapté pour former sur ledit détecteur (12) une image d'une troisième face (9c) du matériau holographique dynamique opposée à ladite première face.

16. Installation selon la revendications 15, dans laquelle lesdites première et deuxième faces (9a) de matériau holographique dynamique sont confondues.

17. Installation selon la revendication 15, dans laquelle ladite deuxième face (9b) de matériau holographique dynamique est orthogonale à ladite première face (9a).

18. Installation selon l'une quelconque des revendications 13 à 17, dans laquelle le dispositif de génération comprend
un laser (6) adapté pour émettre une onde lumineuse initiale,
un dispositif de séparation (7) adapté pour générer à partir de ladite onde initiale un premier faisceau et un deuxième faisceau,
un premier dispositif de décalage adapté pour décaler en fréquence ledit premier faisceau pour générer ladite onde incidente, et
un deuxième dispositif de décalage adapté pour décaler en fréquence ledit deuxième faisceau pour générer ladite onde pompe,
lesdits premier et deuxième dispositifs de décalage étant adaptés pour que ladite onde pompe présente une fréquence sensiblement égale à la somme de la fréquence de l'onde incidente et de la fréquence de l'onde acoustique.

19. Installation selon la revendication 18, dans lequel ledit premier dispositif de décalage comprend un, zéro, ou deux modulateurs acousto-optiques, et dans lequel ledit deuxième dispositif de décalage comprend, respectivement, un, deux, ou zéro modulateurs acousto-optiques.

20. Installation selon l'une quelconque des revendications 13 à 19, dans laquelle ledit matériau holographique dynamique est un cristal photo réfractif.

21. Installation selon l'une quelconque des revendications 13 à 19, dans laquelle ledit matériau holographique dynamique est un matériau à inversion de population apte à être gravé par un réseau d'indice complexe sous forme d'un réseau de gain, ladite installation comprenant en outre une source d'énergie adaptée pour maintenir ladite inversion de population dans ledit matériau.

22. Installation selon l'une quelconque des revendications 13 à 21 comprenant en outre un dispositif de commande adapté pour faire déplacer ledit transducteur (2) et/ou changer la focale dudit transducteur.

## Patentansprüche

1. Akustisch-optisches Bildgebungsverfahren eines Objekts, umfassend die Schritte, im Verlauf derer:
(a) die Vibration eines Bereichs (5) des Objekts (1) per Anwendung einer eine bestimmte akustische Frequenz aufweisenden akustischen Welle auf diesen Bereich angewendet wird;
(b) auf das genannte Objekt eine einfallende Lichtwelle (INC) angewendet wird, die somit eine Signallichtwelle (SIG) generiert, die wenigstens eine akustisch-optische Komponente umfasst, die in der Frequenz durch die genannte akustische Welle verschoben ist,
(c) eine Lichtpumpenwelle (PMP) generiert wird, die mit der genannten, in einer Frequenz einfallenden Welle (IC) kohärent ist, die der Frequenz der genannten akustisch-optischen Komponente ähnlich ist,
(d) ein komplexes Indexnetz durch die Interferenz zwischen der Signalwelle und der Pumpenwelle in ein dynamisches holografisches Material (9) graviert wird, indem die genannte Signalwelle und die genannte Pumpenwelle auf das genannte Material angewendet werden, und
(e) ein digitaler Parameter relativ zu der Lichtintensität in diesem Bereich ausgehend von dem genannten komplexen Indexnetz erhalten wird,
in dem im Verlauf des Schritts (e),
(e1) eine gebrochene Welle per Anwendung der genannten Wellenpumpe (PMP) auf dem genannten komplexen Indexnetz generiert wird, und;
(e2) die Interferenz zwischen der genannten gebrochenen Welle und der genannten Signalwelle mithilfe eines Fotodetektors (12) erfasst wird.

2. Verfahren gemäß dem voranstehenden Anspruch, bei dem eine Phasenmodulation auf eine Welle angewendet wird, die aus der akustischen Welle, der Pumpenwelle (PMP) und der einfallenden Welle (INC) ausgewählt ist.

3. Verfahren gemäß Anspruch 2, bei dem die genannte Phasenmodulation auf die akustische Welle angewendet wird.

4. Verfahren gemäß Anspruch 1, bei dem eine Amplitudenmodulation auf eine Welle angewendet wird, die aus der akustischen Welle, der Pumpenwelle (PM) und der einfallenden Welle (INC) ausgewählt ist.

5. Verfahren gemäß Anspruch 4, bei dem die genannte Amplitudenmodulation auf die akustische Welle angewendet wird.

6. Verfahren gemäß irgendeinem der voranstehenden Ansprüche, bei dem die einfallende Welle und die Pumpenwelle generiert werden
- indem eine anfängliche Lichtquelle (INI) mithilfe eines Lasers generier wird,
- die genannte anfängliche Welle in ein erstes Bündel (F1) und in ein zweites Bündel (F2) getrennt werden
- die Frequenz des genannten ersten Bündels einer ersten Frequenzverschiebung verschoben wird, um die einfallende Welle (INC) zu generieren,
- die Frequenz des genannten zweiten Bündels einer zweiten Frequenzverschiebung verschoben wird, um die Pumpenwelle (PMP) zu generieren,
wobei die genannte zweite Frequenzverschiebung deutlich gleich der Summe der ersten Frequenzverschiebung und der akustischen Frequenz ist.

7. Verfahren gemäß Anspruch 6, bei dem eine der genannten ersten und zweiten Frequenzverschiebungen null ist.

8. Verfahren gemäß irgendeinem der voranstehenden Ansprüche, bei dem im Verlauf des Schritts (d) das genannte Wellensignal auf eine erste Seite (9a) des genannten dynamischen holografischen Materials und die genannte Wellenpumpe auf eine zweite Seite (9) des genannten Materials, das von der ersten Seite unterschiedlich ist, angewendet wird.

9. Verfahren gemäß irgendeinem der Ansprüche 1 bis 7, bei dem im Verlauf des Schritts (d) die genannte Signalwelle und die genannte Pumpenwelle auf eine und dieselbe Seite des dynamischen holografischen Materials angewendet werden.

10. Verfahren gemäß einem der voranstehenden Ansprüche, bei dem das genannte dynamische holografische Material ein fotostrahlender Kristall ist, der für einen Betrieb im Modus "Energieübertragung" geeignet ist.

11. Verfahren gemäß einem der Ansprüche 1 bis 9, bei dem das genannte dynamische holografische Material ein fotostrahlender Kristall ist, der für einen Betrieb im Modus "anisotrope Diffraktion" geeignet ist.

12. Verfahren gemäß irgendeinem der voranstehenden Ansprüche, in dessen Verlauf darüber hinaus eine digitale Information relativ zu einem zweiten Bereich des Objekts unter Anwendung der folgenden Schritte erhalten wird:
(a') Erzeugen der Vibration des genannten zweiten Bereichs unter Anwendung einer akustischen Welle mit einer akustischen Frequenz auf das Objekt an dem genannten zweiten Bereich und
Wiederholen der Schritte (b) bis (e) für den genannten zweiten Bereich.

13. Akustisch-optische Bildgebungsanlage eines Objekts, umfassend
(A) einen Wandler (2), der zum Generieren der Vibration eines Bereichs (5) des Objekts (1) unter Anwendung einer eine akustische Frequenz aufweisenden akustischen Welle auf diesen Bereich des Objekts geeignet ist
(B) eine Vorrichtung zum Generieren von Lichtquellen, die zur Anwendung einer einfallenden Lichtwelle (INC) auf das genannte Objekt geeignet ist, und somit Generieren einer Signallichtquelle (SIG), die wenigstens eine akustisch-optische Komponente umfasst, die in der Frequenz durch die genannte akustische Welle verschoben und darüber hinaus geeignet ist, um eine Lichtpumpenwelle (PMP) zu generieren, die mit der genannten einfallenden Welle mit einer ähnlichen Frequenz wie der genannten akustisch-optischen Komponente kohärent ist,
(C) ein dynamisches holografisches Material (9), das dazu geeignet ist, dass darauf ein komplexes Indexnetz per Interferenz zwischen der Signalwelle und der Pumpenwelle dank der Anwendung der genannten Signalwelle und der genannten Pumpenwelle auf das genannte Material graviert wird und
(D) eine Detektionsvorrichtung, die für den Erhalt eines digitalen Parameters relativ zur Lichtintensität in diesem Bereich ausgehend von dem genannten komplexen Indexnetz geeignet ist,
bei dem die genannte Detektionsvorrichtung (E) einen Detektor (12) umfasst, der zum Detektieren der Interferenz zwischen einer gebeugten Welle, die durch die genannte das genannte komplexe Indexnetz durchquerende Wellenpumpe generiert wird, und die genannte Signalwelle generiert wird.

14. Anlage gemäß Anspruch 13, bei der der genannte Detektor eine Monopixel-Fotodiode ist.

15. Anlage gemäß Anspruch 13 oder Anspruch 14, umfassend:
eine erste optische Vorrichtung (10, 11), die geeignet ist, um die Signalwelle auf eine erste Seite des dynamischen holografischen Materials anzuwenden,
eine zweite optische Vorrichtung (16) die geeignet ist, um die Pumpenwelle auf eine zweite Seite des fotostrahlenden Materials anzuwenden, und
eine dritte optische Vorrichtung (13, 14, 15), die geeignet ist, um auf dem genannten Detektor (12) ein Bild einer dritten Seite (9c) des dynamischen holografischen Materials gegenüber der genannten ersten Seite zu bilden.

16. Anlage gemäß Anspruch 15, bei der die genannte erste und zweite Seite (9a) des dynamischen holografischen Materials zusammenfallen.

17. Anlage gemäß Anspruch 15, bei der die genannte zweite Seite (9b) des dynamischen holografischen Materials orthogonal zu der genannten ersten Seite (9a) ist.

18. Anlage gemäß irgendeinem der Ansprüche 13 bis 17, bei der die Vorrichtung zum Generieren umfasst
einen Laser (6), der zum Ausgeben einer anfänglichen Lichtwelle geeignet ist,
eine Trennvorrichtung (7), die zum Generieren eines ersten Bündels und eines zweiten Bündels ausgehend von der genannten anfänglichen Welle geeignet ist,
eine erste Versetzungsvorrichtung, die für die Frequenzverschiebung des genannten ersten Bündels zum Generieren der genannten einfallenden Welle geeignet ist, und
eine zweite Versetzungsvorrichtung, die zur Frequenzverschiebung des genannten zweiten Bündels zum Generieren der genannten Pumpenwelle geeignet ist,
wobei die genannte erste und zweite Versetzungsvorrichtung dazu geeignet sind, dass die genannte Pumpenwelle eine deutlich gleiche Frequenz aufweist wie die Summe der Frequenz der einfallenden Welle und der Frequenz der akustischen Welle.

19. Anlage gemäß Anspruch 18, bei der die genannte erste Versetzungsvorrichtung einen, null oder zwei akustisch-optische Modulatoren umfasst und bei dem die genannte zweite Versetzungsvorrichtung jeweils einen, zwei oder null akustisch-optische Moderatoren umfasst.

20. Anlage gemäß irgendeinem der Ansprüche 13 bis 19, bei der das genannte dynamische holografische Material ein fotostrahlender Kristall ist.

21. Anlage gemäß irgendeinem der Ansprüche 13 bis 19, bei der das genannte dynamische holografische Material ein Material mit Populationsinversion ist, das geeignet ist, durch ein komplexes Indexnetz in Form eines Verstärkungsnetzes geätzt zu werden, wobei die genannte Anlage darüber hinaus eine Energiequelle umfasst, die geeignet ist, um die genannte Populationsinversion in dem genannten Material zu halten.

22. Anlage gemäß irgendeinem der Ansprüche 13 bis 21, umfassend darüber hinaus eine Steuervorrichtung, die geeignet ist, um den genannten Wandler (2) versetzen zu lassen und/oder die Brennweite des genannten Wandlers zu ändern.

## Claims

1. A method for acousto-optically imaging an object comprising the steps of:
(a) generating the vibration of a zone (5) of the object (1) by applying on this zone of the object, an acoustic wave having some acoustic frequency,
(b) applying to said object an incident light wave (INC) thus generating a signal light wave (SIG) comprising at least one acousto-optical component frequency-offset due to said acoustic wave,
(c) generating a light pump wave (PMP), coherent with said incident wave (INC) at a frequency similar to the frequency of said acousto-optical component,
(d) etching a complex index network in a dynamic holographic material (9) by the interference between the signal wave and the pump wave by applying to said material said signal wave and said pump wave, and
(e) obtaining a numerical parameter relating to the light intensity in this zone from said complex index network, comprising, during step (e),
(e1) generating a diffracted wave by applying said pump wave (PMP) on said complex index network, and
(e2) detecting the interference between said diffracted wave and said signal wave using a photodetector (12).

2. The method according to the preceding claim, wherein a phase modulation is applied to a wave chosen from the acoustic wave, the pump wave (PMP), and the incident wave (INC).

3. The method according to claim 2, wherein said phase modulation is applied to the acoustic wave.

4. The method according to claim 1, wherein an amplitude modulation is applied to a wave chosen from the acoustic wave, the pump wave (PMP) and the incident wave (INC).

5. The method according to claim 4, wherein said amplitude modulation is applied to the acoustic wave.

6. The method according to any of the preceding claims, wherein the incident wave and the pump wave are generated by
- generating an initial light wave (INI) using a laser,
- separating said initial wave into a first beam (F1) and a second beam (F2),
- offsetting the frequency of said first beam by a first frequency offset to generate the incident wave (INC),
- offsetting the frequency of said second beam by a second frequency offset to generate the pump wave (PMP),
said second frequency offset being substantially equal to the sum of the first frequency offset and the acoustic frequency.

7. The method according to claim 6, wherein one of said first and second frequency offsets is null.

8. The method according to any of the preceding claims, wherein, during step (d), said signal wave is applied to a first face (9a) of said dynamic holographic material and said pump wave to a second face (9b) of said material, distinct from the first face.

9. The method according to any of claims 1 to 7, wherein, during step (d), said signal wave and said pump wave are applied to a same face of the dynamic holographic material.

10. The method according to one of the preceding claims, wherein said dynamic holographic material is a photo refractive crystal adapted to operate in an "energy transfer" mode.

11. The method according to one of claims 1 to 9, wherein said dynamic holographic material is a photo refractive crystal adapted to operate in an "anisotropic diffraction" mode.

12. The method according to any of the preceding claims, during which a numerical piece of information relating to a second zone of the object is further obtained by applying the following steps of:
(a') generating the vibration of said second zone by applying to the object at said second zone an acoustic wave at an acoustic frequency, and
repeating steps (b) to (e) for said second zone.

13. A facility for acousto-optically imaging an object comprising
(A) a transducer (2) adapted to generate the vibration of a zone (5) of the object (1) by applying to this zone of the object an acoustic wave having an acoustic frequency,
(B) a device for generating light waves adapted to apply to said object an incident light wave (INC) and thus generating a signal light wave (SIG) including at least one acousto-optical component frequency-offset by said acoustic wave, and further adapted to generate a light pump wave (PMP), coherent with said incident wave at a frequency similar to the frequency of said acousto-optical component,
(C) a dynamic holographic material (9) adapted such that a complex index network is etched therein by the interference between the signal wave and the pump wave, by virtue of applying to said material said signal wave and said pump wave, and
(D) a detecting device adapted to obtain a numerical parameter relating to the light intensity at this zone from said complex index network, wherein said detecting device (E) comprises a detector (12) adapted to detect the interference between a diffracted wave generated by said pump wave passing through said complex index network, and said signal wave.

14. The facility according to claim 13, wherein said detector is a monopixel photodiode.

15. The facility according to claim 13 or claim 14, comprising
a first optical device (10, 11) adapted to apply the signal wave to a first face of the dynamic holographic material,
a second optical device (16) adapted to apply the pump wave to a second face of the photo refractive material, and
a third optical device (13, 14, 15) adapted to form on said detector (12), an image of a third face (9c) of the dynamic holographic material opposed to said first face.

16. The facility according to claim 15, wherein said first and second faces (9a) of the dynamic holographic material are identical.

17. The facility according to claim 15, wherein said second face (9b) of dynamic holographic material is orthogonal to said first face (9a).

18. The facility according to any of claims 13 to 17, wherein the generating device comprises
a laser (6) adapted to emit an initial light wave,
a separating device (7) adapted to generate from said initial wave a first beam and a second beam,
a first offset device adapted to frequency-offset said first beam to generate said incident wave, and
a second offset device adapted to frequency-offset said second beam to generate said pump wave,
said first and second offset devices being adapted such that said pump wave has a frequency substantially equal to the sum of the frequency of the incident wave and the frequency of the acoustic wave.

19. The facility according to claim 18, wherein said first offset device comprises one, zero, or two acousto-optical modulators, and wherein said second offset device comprises one, two, or zero acousto-optical modulators respectively.

20. The facility according to any of claims 13 to 19, wherein said dynamic holographic material is a photo refractive crystal.

21. The facility according to any of claims 13 to 19, wherein said dynamic holographic material is a population inversion material able to be etched by a complex index network as a gain network, said facility further comprising an energy source adapted to maintain said population inversion in said material.

22. The facility according to any of claims 13 to 21, further comprising a control device adapted to move said transducer (2) and/or change the focal distance of said transducer.
